Europäisches Patentamt

European Patent Office

Office européen des brevets

(11) Publication number: **0 159 777**
A1

# EUROPEAN PATENT APPLICATION

(21) Application number: 85301164.1

(22) Date of filing: 21.02.85

(51) Int. Cl.⁴: **A 61 K 31/415,** A 61 K 47/00

(30) Priority: 05.03.84 JP 43265/84

(43) Date of publication of application: 30.10.85
Bulletin 85/44

(84) Designated Contracting States: **AT BE CH DE FR GB IT LI NL SE**

(71) Applicant: SUMITOMO PHARMACEUTICALS COMPANY, LIMITED, 40, Dosho-machi 2-chome, Higashi-ku Osaka-shi Osaka-fu (JP)

(72) Inventor: Kato, Keiichi, 2-29-7, Oike, Ibaraki Osaka-fu (JP)
Inventor: Hara, Muneo, 8-6-11, Fukushima, Fukushima-ku Osaka (JP)
Inventor: Tobiki, Hisao, 4-12-308, Konancho 2-chome, Higashinada-ku Kobe Hyogo-ken (JP)

(74) Representative: Geering, Keith Edwin et al, REDDIE & GROSE 16 Theobalds Road, London WC1X 8PL (GB)

(54) Stabilized 4-carbamoyl-imidazolium-5-olate.

(57) This invention provides a pharmaceutical composition containing medicament selected from 4-carbamoyl-imidazolium-5-olate and salts and hydrates thereof stabilized against discoloration by the presence of acid substance having a dissociation constant of 4 or less.

<u>STABILIZED 4-CARBAMOYL-IMIDAZOLIUM-5-OLATE</u>

The present invention relates to a method for stabilizing preparations containing 4-carbamoyl-imidazolium-5-olate or its salt or hydrate thereof (hereinafter referred to as "the compound") as an active ingredient, the stabilized preparations and a method for the production thereof.

The compound is a well-known one and known to have a strong carcinostatic activity (Canadian Patent No.1,078,736).

The compound has the property of coloring easily on exposure to oxygen, heat or light, and when the compound is formulated, for example into oral administration preparations, it is observed that the coloration of the compound tends to become more marked by the interaction of the compound with the coexisting vehicles accompanied by complicated reactions.

The conventionally well known method to prevent this coloration in designing preparations is to use sulfur compounds producing $HSO_3^-$, $SO_3^{2-}$ or $S_2O_5^{2-}$ ions as a stabilizing agent as disclosed in EP Patent Application No.81305148.9. These sulfur compounds display strong power in preventing the coloration of the compound, but when preparations containing them are taken, their peculiar offensive odor is felt so that they are not always satisfactory for producing solid preparations for oral administration.

After extensive search for substances pharmaceutically acceptable as additives for the compound, we have found that the coexistence of acid substance with the compound can significantly resist its coloration in various preparations including solid preparations.

As acid substances for use in the present invention, those having a dissociation constant (pka) of 4 or less are suitable. For example, there are given inorganic acids (e.g. phosphoric acid, hydrochloric acid), organic acids (e.g. citric acid, tartaric acid, malic acid, fumaric acid, oxalic acid) and acid coating agents [e.g. methacrylic acid/ethyl acrylate copolymer (Eudragit $^{®}$ L30D-55)] and the like.

A suitable amount of coexisting acid substance is e.g. 0.001 to 0.5 mole per mole of the compound, and amounts of 0.005 to 0.2 m/m are preferred. When methacrylic acid/ethyl acrylate copolymer is used, amounts of 0.01 to 0.5 parts w/w based on the compound are suitable.

The preparation of the present invention can be produced as follows. For example, solid preparations can be produced by mixing the compound, acid substance and if necessary, appropriate amount of pharmaceutically acceptable vehicle, binding agent, disintegrator, lubricant, etc. and formulating the resulting mixture into tablets, granular preparations, capsules and the like by the usual methods. In this case, for making the acid substance coexist with the compound, the following methods may be used : a method in which the compound, vehicles, disintegrators, etc. and    acid substance are mixed together in a powdery state to produce a powder for capsules, or the resulting mixture is dry-granulated or wet-granulated; and a method in which the acid substance, in a liquid state or                              dissolved or dispersed in combination with a binding agent in a solvent, is mixed with the compound, vehicles, disintegrators and the like to produce liquid preparations, or the resulting mixture is wet-granulated.

Suitable vehicles include  for example starches (e.g. corn starch, potato starch), saccharides (e.g. milk sugar, cane sugar, grape sugar, mannitol) and the like.

Suitable binding agents include  for example starches (e.g. corn starch, potato starch), cellulose derivatives (e.g. methyl cellulose, ethyl cellulose, hydroxypropyl cellulose), synthetic high polymers ( e.g. polyvinyl alcohol, polyvinyl pyrrolidone) and the like.

Suitable disintegrators include  for example starches (e.g. corn starch, potato starch), carboxymethyl cellulose or its calcium salt, low-substituted hydroxypropylmethyl cellulose and the like.

Suitable lubricants include  for example magnesium stearate, calcium stearate, stearic acid, talc and the like.

The application of the preparation of the present invention is not limited to solid preparations,       but preparations such as injections, syrups, emulsions, ointments, creams and suppositories can also be produced by mixing the compound,       acid substance and

if necessary, pharmaceutically usable dissolution-assistants, isotonic agents, antiseptic agents, buffers, pH-regulators, thickening agents, etc. and formulating the resulting mixture by the usual methods.

The usefulness of the present invention is illustrated by the following experimental examples and examples.

Experimental example 1

4-Carbamoyl-imidazolium-5-olate hydrate was dissolved in purified water so that its concentration was 0.02 mole/liter, and to this aqueous solution was added an organic acid ( 1/100 m/m based on said hydrate ). 20 Milliliters of this solution was tight sealed in a glass bottle and stored at 50°C for 4 days, and the degree of coloration was visually observed. The results for various acids are shown in Table 1.

Table 1

| Sample | Color |
|---|---|
| Control (no addition of organic acid) | Bluish violet |
| Fumaric acid (pka=3.02) | Colorless |
| Tartaric acid (pka=3.04) | Colorless |
| Citric acid (pka=3.13) | Colorless |
| dℓ-Malic acid (pka=3.46) | Colorless |
| Ascorbic acid (pka=4.17) | Pale blue |
| Succinic acid (pka=4.21) | Pale blue |

Experimental example 2

To 30 g of 4-carbamoyl-imidazolium-5-olate hydrate was added 10 g of a 30% aqueous solution of methacrylic acid/ethyl acrylate copolymer (Eudragid®L30D-55), and the resulting mixture was thoroughly mixed, dried, sieved and formulated into granules. The granules thus obtained were packed in a glass bottle and stored under the following conditions and the degree of coloration of the granules was observed to obtain the results in Table 2.   For comparison, granular  4-carbamoyl-imidazolium-5-olate hydrate alone (Comparative example) was similarly stored, and the degree of coloration was observed.

<div align="center">Table 2</div>

| Storage conditions | | Experimental example | Comparative example |
|---|---|---|---|
| 40°C | 1 month | — | + |
| 40°C ✕ 75% RH (relative humidity) | 1 month | — | + |
| 50°C | 15 days | — | ++ |

Standard for evaluation (same applies also to the following examples):

—  : No coloration

±  : Colored slightly greenish blue

+  : Colored greenish blue

++ : Colored strongly greenish blue

Example 1

Granules :

Recipe

|  | mg/gram granule |
|---|---|
| 4-Carbamoyl-imidazolium-5-olate hydrate | 400 |
| Milk sugar | 450 |
| Corn starch | 148 |
| Citric acid | 2 |

(Molar ratio of citric acid to 4-carbamoyl-imidazolium-5-olate hydrate, 0.01 : 1)

4-Carbamoyl-imidazolium-5-olate hydrate, milk sugar and corn starch were mixed on a universal mixer, and a corn starch paste containing dissolved citric acid was added thereto. The resulting mixture was kneaded and granulated through an extrusion-granulator (screen diameter, 0.8 mmφ), dried and made uniform in size to obtain granules.

Storage test

The granules prepared as above were packed in a glass bottle and stored under the following conditions, and the degree of coloration of the granules was observed to obtain the results in Table 3. For comparison, granules prepared without adding citric acid in the above recipe (comparative example) was similarly stored, and the degree of coloration was observed.

Table 3

| Storage conditions | | Example | Comparative example |
|---|---|---|---|
| 40°C | 1 month | — | — |
| 40°C × 75% RH | 1 month | — | + |

Example 2

Tablet :

Recipe

|  | mg/tablet |
|---|---|
| 4-Carbamoyl-imidazolium-5-olate hydrate | 200 |
| Milk sugar | 10 |

- 7 -

0159777

| Corn starch | 13 |
| Carboxymethyl cellulose calcium | 10 |
| Polyvinyl alcohol | 10 |
| Magnesium stearate | 2 |
| Citric acid | 5 |

(Molar ratio of citric acid to 4-carbamoyl-imidazolium-5-olate hydrate, 0.05 : 1)

4-Carbamoyl-imidazolium-5-olate hydrate, milk sugar, corn starch, carboxymethyl cellulose calcium and citric acid were mixed on a universal mixer, and an aqueous polyvinyl alcohol solution was added thereto. The resulting mixture was kneaded, pulverized on a power mill, dried and made uniform in size. The powder thus obtained was mixed with magnesium stearate and compression-molded into tablets having a weight of 250 mg/tablet and a diameter of 9 mm.

Storage test

The tablets prepared as above were packed in a glass bottle and stored under the following conditions, and the degree of coloration of the tablets was observed to obtain the results in Table 4. For comparison, a tablet prepared without adding citric acid in the above recipe (comparative example) was similarly stored, and the degree of coloration was observed.

Table 4

| Storage conditions | | Example | Comparative example |
| --- | --- | --- | --- |
| 40°C | 1 month | — | + |
| 50°C | 15 days | — | + |

Example 3

4-Carbamoyl-imidazolium-5-olate hydrate was dissolved in purified water so that its concentration was 0.02 mole/liter, and to this solution was added an acid (1/100 m/m based on said hydrate). 20 Milliliters of this solution was tightly sealed in a glass bottle and stored at 50°C for 4 days, and the degree of coloration was visually observed. The results were as shown in Table 5.

Table 5

| . Sample | Color |
|---|---|
| Control (no addition of inorganic acid) | Bluish violet |
| Hydrochloric acid | Colorless |
| Phosphoric acid (pka=2.15) | Colorless |
| Acetic acid (pka=4.76) | Pale yellow |

As apparent from Experimental examples 1, 2 and 3 and Examples 1 and 2 above, the coloration of preparations containing 4-carbamoyl-imidazolium-5-olate during storage could be prevented by making the acid substance coexist in the preparations.

## C L A I M S :

1. A pharmaceutical composition containing medicament selected from 4-carbamoyl-imidazolium-5-olate and salts and hydrates thereof stabilized against discoloration by the presence of acid substance having a dissociation constant of 4 or less.

2. A composition according to claim 1 wherein the acid substance is selected from phosphoric and hydrochloric acids.

3. A composition according to claim 1 wherein the acid substance is selected from citric, tartaric, malic and fumaric acids.

4. A composition according to claim 1 wherein the acid substance is an acid coating agent.

5. A composition according to claim 1, 2 or 3 containing 0.001 to 0.5 mole of said acid substance per mole of said medicament.

6. A method for stabilizing a preparation comprising medicament selected from 4-carbamoyl-imidazolium-5-olate and salts and hydrates thereof, the method comprising mixing or coating the preparation with acid substance having a dissociation constant of 4 or less.

7. A method according to claim 6 wherein said medicament and acid substance are mixed in a powdery state.

0159777

8.   A method according to claim 6 wherein said medicament is mixed with said acid substance which is in a liquid state or dissolved or dispersed in a liquid.

# 0159777

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 051 962 (SUMITOMO CHEMICAL COMPANY, LTD.) * Page 14, lines 1-18; claims 1-6 * | 1-8 | A 61 K 31/415 A 61 K 47/00 |
| | --- | | |
| D,A | US-A-4 181 731 (YOSHIDA) * & CA - A - 1 078 736 * | 1-8 | |
| | ----- | | |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 12-06-1985 | BRINKMANN C. |